# EUROPEAN PATENT APPLICATION

(11) **EP 4 755 441 A1**
(43) Date of publication of application: **10.06.2026**
(21) Application number: 25220344.3
(22) Date of filing: 03.12.2025
(51) Int. Cl.: A61N 1/05

(54) **IMPLANTABLE LEAD**

(30) Priority: 06.12.2024 US 202463728839 P; 02.12.2025 US 202519405927
(71) Applicant: Pacesetter, Inc., Sylmar, CA 91342 (US)
(72) Inventor: Ledbetter, Cody, Sylmar, 91342 (US); Alleman, Wesley, Sylmar, CA 91342 (US); James IV, Benjamin F, Sylmar, CA 91342 (US)
(74) Representative: Barker Brettell Sweden AB

(57) **Abstract**

An implantable lead (400) is described that includes a header coupling (200), a helix shaft (302), and a fixation helix (106). The header coupling (200) extends from a proximal end (204) to a distal end (206) thereof, and defines a central lumen (208) from the proximal end (204) to the distal end (206). The header coupling (200) includes a base section (212) and a distal tube section (214) extending from the base section (212) to the distal end (206). The helix shaft (302) includes a proximal shank (306) that extends into the central lumen (208) of the header coupling (200) through a distal opening (210) of the distal tube section (214). The fixation helix (106) is mounted to a distal segment (304) of the helix shaft (302) and is configured to penetrate tissue of a patient. A surface (222) of the header coupling (200) directly engages and is fixedly secured to a surface (312) of the helix shaft (302), forming a sealed joint (308) that seals the central lumen (208) of the header coupling (200) and prevents fluid from migrating past the distal end (206).

## Description

### TECHNICAL FIELD

Embodiments of the present disclosure relate generally to implantable leads, and more specifically to implantable leads of medical devices within a patient.

### BACKGROUND

Some implantable medical devices (IMDs) function to monitor cardiac activity of a patient, provide electrotherapy to the cardiac tissue, and/or the like. Some cardiac pacemakers and implantable cardioverter-defibrillators (ICD) use insulated wires called implantable leads, implantable cardiac leads, or simply leads to monitor the heart and provide stimulation therapy by delivering electrical pacing pulses and/or shocks. Some leads pierce the myocardial tissue of the heart to deliver stimulation therapy directly into the tissue. Some leads include a central lumen (e.g., channel) that allows delivery of another tool through the lead body. The other tool can be a stylet that is used for maneuvering the lead during implant and/or extracting the lead during explant (e.g., lead removal). For example, a locking stylet may be inserted from a proximal end of the lead through the central lumen. The stylet can provide structural support and maneuverability for the lead during implant and/or explant procedures. During explant, a stylet can be inserted through the central lumen for the purpose of gaining traction of an inner coil within the lead.

It is desirable to keep the central lumen of the lead open and unobstructed to permit full insertion of stylets and other tools into the lead. For example, if a stylet is not able to reach a threshold proximity to the distal end of the lead, sections of the lead may be compromised as the stylet applies traction on the lead. For example, a portion of the lead distal to the stylet may stretch and fracture (e.g., break), which may substantially impede and complicate the explant procedure.

The central lumen of the lead may become obstructed due to the presence of organic tissue from the patient penetrating the central lumen. For example, blood may seep into the central lumen while the lead is in the patient body. The blood in the central lumen may coagulate and harden over time, which can restrict or prevent stylets and other tools from reaching desired distances into the lead. As a consequence, the lead explant procedure can experience one or more undesirable effects, such as limited traction and/or enhanced risk of lead damage (e.g., fracture).

There is a need for an implantable lead designed to seal the central lumen to prevent the inflow of blood and other patient tissue into the central lumen, thereby maintaining the central lumen open and unobstructed for accommodating stylets and other tools.

### SUMMARY

In accordance with embodiments herein, an implantable lead is provided that includes a header coupling, a helix shaft, and a fixation helix. The header coupling extends from a proximal end of the header coupling to a distal end of the header coupling. The header coupling defines a central lumen that extends from the proximal end to the distal end. The header coupling includes a base section and a distal tube section extending from the base section to the distal end. The helix shaft includes a proximal shank that extends into the central lumen of the header coupling through a distal opening of the distal tube section. The fixation helix is mounted to a distal segment of the helix shaft and configured to penetrate tissue of a patient. A surface of the header coupling directly engages and is fixedly secured to a surface of the helix shaft, forming a sealed joint that seals the central lumen of the header coupling and prevents fluid from migrating past the distal end.

In accordance with embodiments herein, an implantable lead is provided that includes a header coupling, a helix shaft, and a fixation helix. The header coupling extends from a proximal end of the header coupling to a distal end of the header coupling. The header coupling defines a central lumen that extends from the proximal end to the distal end. The header coupling includes a base section and a distal tube section extending from the base section to the distal end. The helix shaft includes a proximal shank that extends into the central lumen of the header coupling through a distal opening of the distal tube section. The fixation helix is mounted to a distal segment of the helix shaft and configured to penetrate tissue of a patient. The distal tube section of the header coupling is crimped onto the proximal shank of the helix shaft, forming a crimp joint that seals the central lumen of the header coupling and prevents fluid from migrating past the distal end and into a central lumen of the lead.

In accordance with embodiments herein, a method is provided for assembling a distal end segment of an implantable lead. The method includes obtaining a header coupling that extends from a proximal end of the header coupling to a distal end of the header coupling. The header coupling defines a central lumen that extends from the proximal end to the distal end. The header coupling includes a base section and a distal tube section extending from the base section to the distal end. The method includes loading a proximal shank of a helix shaft into the central lumen of the header coupling through a distal opening of the distal tube section. The method includes mounting a fixation helix to a distal segment of the helix shaft. The fixation helix penetrates tissue of a patient. The method includes forming a sealed joint between a surface of the header coupling and a surface of the helix shaft to seal the central lumen of the header coupling and to prevent fluid from migrating past the distal end. The surface of the header coupling directly engages and is fixedly secured to the surface of the helix shaft at the sealed joint.

In accordance with embodiments herein, an implantable lead is provided that includes a header coupling, a helix shaft, and a fixation helix. The header coupling extends from a proximal end of the header coupling to a distal end of the header coupling. The header coupling defines a central lumen that extends from the proximal end to the distal end. The helix shaft includes a proximal shank that extends into the central lumen of the header coupling. The fixation helix is mounted to a distal segment of the helix shaft and configured to penetrate tissue of a patient. A surface of the header coupling directly engages and is fixedly secured to a surface of the helix shaft, forming a sealed joint that seals the central lumen of the header coupling and prevents fluid from migrating past the distal end and into a central lumen of the lead. The sealed joint is one of (i) a press-fit mating interface between an inner surface of the header coupling that defines the central lumen and an outer surface of the helix shaft, or (ii) a weld joint between the surface of the header coupling and the surface of the helix shaft.

### BRIEF DESCRIPTION OF THE DRAWINGS

Figure 1 illustrates a schematic cutaway view of a heart relative to an implantable system comprising an IMD and an implantable lead.
Figure 2 is another schematic cutaway view of the heart showing a location of the bundle of His (e.g., His bundle) in the heart.
Figure 3 illustrates an implantable lead according to an embodiment.
Figure 4 is an elevation view of a header coupling of an implantable lead according to an embodiment.
Figure 5 is a cross-sectional view of the header coupling of Figure 4.
Figure 6 is a partial cross-sectional view of a header assembly of an implantable lead according to an embodiment.
Figure 7 is a partial cross-sectional view of a distal end segment of an implantable lead according to an embodiment.
Figure 8 is a close-up, partial cross-sectional view of the header assembly shown in Figures 6 and 7 according to a second embodiment.
Figure 9 is a close-up, partial cross-sectional view of the header assembly according to a third embodiment.
Figure 10 is a close-up, partial cross-sectional view of the header assembly according to a fourth embodiment.
Figure 11 is a flow chart of a method for assembling a distal end segment of an implantable lead according to an embodiment.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or implantable leads and/or IMDs) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more IMDs, devices, or systems. While the operations of some methods may be described as performed by the processor(s) of one device, additionally, some or all of such operations may be performed by the processor(s) of another device described herein.

Embodiments may be implemented in connection with one or more implantable medical devices (IMDs). Non-limiting examples of IMDs include neurostimulator therapy devices, such as a cardiac monitoring device, pacemaker, cardioverter, cardiac rhythm management device, defibrillator, neurostimulator, and the like. For example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System".

Embodiments set forth herein describe implantable leads that are designed to have an integrated blood seal. More specifically, the implantable leads described herein include a distal portion designed to prevent blood and other organic fluid from entering a central lumen of the implantable lead. The distal portion of the lead includes, in part, a header coupling (e.g., a header coupling member) and a helix shaft. The header coupling defines a central lumen, which represents a distal section of the central lumen of the implantable lead. At least a portion of the helix shaft extends into the central lumen of the header coupling. The helix shaft is mounted to a fixation helix that is designed to penetrate tissue of the patient to secure the distal end of the lead to the patient. The integrated blood seal is defined by a sealed joint between a surface of the header coupling and a surface of the helix shaft. In various embodiments, the sealed joint is form by direct contact (e.g., engagement) between the respective surfaces of the two components. The sealed joint fixedly secures the header coupling to the helix shaft. For example, once the sealed joint is formed, the header coupling is rigidly fixed to the helix shaft. The header coupling may not be able to rotate or translate relative to the helix shaft.

In a first embodiment, the sealed joint is a crimp joint. For example, a distal tube segment of the header coupling may be circumferentially crimped onto a shank of the helix shaft to achieve the sealed joint. The crimping operation may cause the distal tube section to have an annular imprint (e.g., deformation) along an outer surface of the distal tube section. The annular imprint is attributable to a crimping tool.

In a second embodiment, the sealed joint is a weld joint, so that the header coupling is welded to the helix shaft. For example, a distal end of the header coupling may be welded to a proximal face of an intermediate flange of the helix shaft. In another example, the distal end of the header coupling may be welded to an outer surface of the helix shaft, such as the cylindrical outer surface of a shank of the helix shaft. In yet another example, a proximal end of the header coupling may be welded to the outer surface of the helix shaft to form the sealed joint.

In a third embodiment, the sealed joint is defined by a press-fit mating interface between an inner surface of the header coupling and an outer surface of the helix shaft. For example, a shank of the helix shaft may taper in a proximal direction along a mating section of the proximal shank. The tapered mating section forms the press-fit interface with the inner surface of the header coupling when the shank of the helix shaft is inserted into the central lumen of the header coupling through a distal opening of the header coupling.

In general, the implantable leads described herein have the header coupling permanently joined to the helix shaft which seals a central (e.g., inner) lumen of the implantable lead from any blood intrusion. A technical effect of the implantable leads described herein is that the central lumen of the implantable lead will remain accessible and unobstructed during the implanted lifetime. As an example, the central lumen may remain free of coagulated blood and other organic fluid of the patient. With the central lumen open, a physician or other operator may be able to efficiently and effectively remove the implantable lead from the patient when desired or necessary. For example, a locking stylet may be able to reach a desired depth or distance within the central lumen to allow the locking stylet to apply sufficient tensile load (e.g., traction) on an inner coil of the implantable lead to remove the implantable lead without lead fracture. In another example, the torque driving stylet may be able to reach a desired depth or distance within the central lumen to allow the torque driving stylet to apply sufficient torque on the fixation element of the implantable lead and/or the header coupler to facilitate counter-rotation to remove the implantable lead.

In an example application, the implantable lead described herein may be a cardiac lead that is implanted into cardiac tissue of a patient. The implantable lead may be designed to deliver electrical stimulation therapy directly to the cardiac tissue. In an example application, the implantable lead may be used to provide electrical stimulation in the form of conduction system pacing (CSP). CSP is a technique of cardiac pacing that involves implanting a pacing or defibrillation lead into the interventricular septum between the left and right ventricles to reach the left ventricular nerve bundle branch (LBB) of the His bundle of the heart. The systems and/or IMDs described herein optionally may use more than one of the implantable lead described herein.

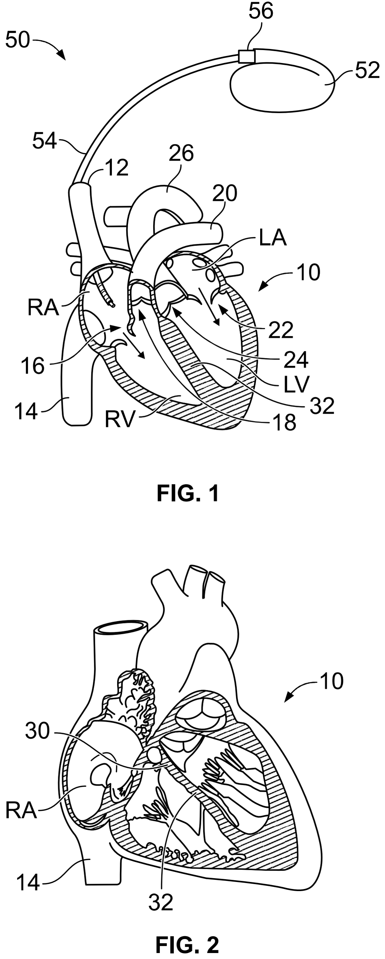

Figure 1 illustrates a schematic cutaway view of a heart 10 relative to an implantable system 50. The heart 10 includes a right atrium RA, a right ventricle RV, a left atrium LA, and a left ventricle LV. During normal operation of the heart 10, deoxygenated blood from the body is returned to the right atrium RA from the superior vena cava 12 and inferior vena cava 14. The right atrium RA pumps the blood through the atrioventricular or tricuspid valve 16 to the right ventricle RV, which then pumps the blood through the pulmonary valve 18 and the pulmonary artery 20 to the lungs for reoxygenation and removal of carbon dioxide. The newly oxygenated blood from the lungs is transported to the left atrium LA, which pumps the blood through the mitral valve 22 to the left ventricle LV. The left ventricle LV pumps the blood through the aortic valve 24 and the aorta 26 throughout the body.

Figure 2 is another schematic cutaway view of the heart 10 showing a location of the bundle of His 30, also referred to as His bundle, in the heart 10. The His bundle 30 consists of fast-conducting muscle fibers that begin at the atrioventricular node in the right atrium and pass to the interventricular septum 32, also referred to as septal wall, between the left and right ventricles. The His bundle 30 divides in the interventricular septum 32 into a right branch and a pair of left branches. The His bundle 30 serves as an initial pathway for electrical impulses to travel from the atria to the ventricles. For example, the His bundle 30 transmits electrical signals from the atrioventricular (AV) node to the right and left bundle branches. The right branch travels along the right side of the interventricular septum 32 and as part of the cardiac conduction system supplies excitation to the right ventricle. The left bundle branch (LBB) travels along the left side of the interventricular septum 32 and supplies excitation (e.g., electrical impulses) to the Purkinje fibers and left ventricle. The LBB facilitates synchronized contraction of the left ventricle. The fibers in the branches terminate in an extensive network of the Purkinje fibers which distribute excitation pulses to the layer of cells beneath the endocardium.

Returning to Figure 1, the implantable system 50 includes an IMD 52 that is operably coupled to an implantable lead 54 through a lead adaptor 56. The lead adaptor 56 is configured to receive a lead connector (not shown) of the implantable lead 54. Although the implantable system 50 includes only one implantable lead in Figure 1, the implantable system 50 may include multiple, i.e., at least two, implantable leads in other embodiments. In an embodiment, the implantable lead 54 is a so-called implantable cardiac lead designed to penetrate the cardiac tissue of the heart 10. In an example, the implantable lead 54 may be positioned and advanced to penetrate the endocardium of the septal wall 32 to reach the LBB of the His bundle 30. The implantable lead 54 may be a transvenous lead that enters the vascular system through one of several possible vascular access sites. For example, the implantable lead 54 may extend through the superior vena cava 12 to the right atrium RA.

The IMD 52 may be a pulse generator device. In one embodiment, the IMD 52 may be a cardiac pacemaker. In other embodiments, however, the IMD 52 may be an intracardiac defibrillator (ICD), a cardiac resynchronization therapy defibrillator (CRT-D), or the implantable system 50 may include an ICD coupled with a pacemaker, and the like. The IMD 52 may be a dual-chamber stimulation device capable of treating both fast and slow arrhythmias with stimulation therapy, including cardioversion, defibrillation, and pacing stimulation, as well as capable of detecting heart failure, evaluating its severity, tracking the progression thereof, and controlling the delivery of therapy and warnings in response thereto. The IMD 52 may be controlled to sense atrial and ventricular waveforms of interest, discriminate between two or more ventricular waveforms of interest, deliver stimulus pulses or shocks, and inhibit application of a stimulation pulse to a heart based on the discrimination between the waveforms of interest and the like. The IMD 52 may provide pacing stimulation to the LBB via the implantable lead 54.

Although not shown, the IMD 52 may wirelessly communicate with an external device, such as a programmer device. The external device may be used by a physician or other technician to select and/or modify therapy parameters to be implemented by the IMD 52, or to bidirectionally communicate information between the IMD 52 and a remote monitoring system (not shown).

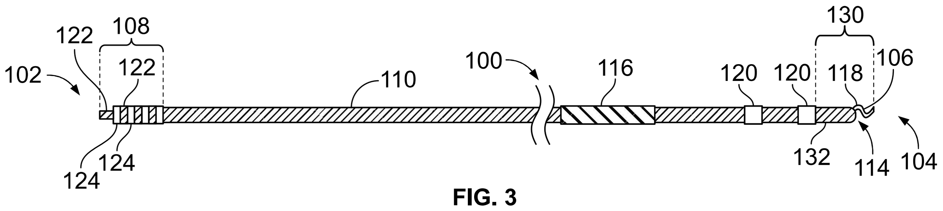

Figure 3 illustrates an implantable lead 100 according to an embodiment. The implantable lead 100 may be the implantable lead 54 shown in Figure 1. The implantable lead 100 extends from a proximal end 102 to a distal end 104. The implantable lead 100 includes a fixation helix 106 at the distal end 104. In an embodiment, the implantable lead 100 includes a terminal connection segment 108 at the proximal end 102. The implantable lead 100 includes a lead body 110 that extends from the terminal connection segment 108 to an electrode at or proximal to the distal end 104. The implantable lead 100 may have a distal end segment 130 at the distal end 104.

The distal end segment 130 includes a fixation helix 106 that projects beyond a distal end 114 of an outer jacket or sleeve 132 of the implantable lead 100. The fixation helix 106 may be a corkscrew designed to be screwed into cardiac tissue. The fixation helix 106 may be composed of a metallic material. A rotational torque applied to the implantable lead 100 may drive the fixation helix 106 into the cardiac tissue, such as the septal wall 32, to engage and worm the implantable lead 100 to the tissue. The fixation helix 106 may anchor the distal end 104 of the implantable lead 100 to the tissue. In embodiments described herein, the fixation helix 106 is fixed in place relative to the lead body 110 and outer jacket 132. For example, the fixation helix 106 permanently projects beyond the distal end 114 of the outer jacket 132.

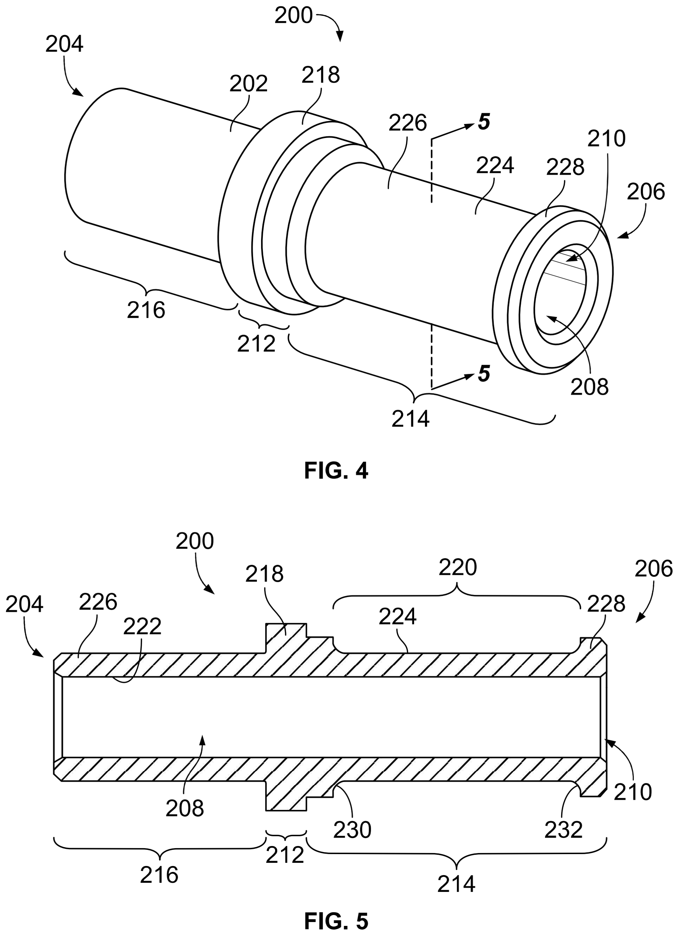

This is in contrast to some known implantable leads which have a selectively extendable fixation helix. The selectively extendable fixation helices may be fully nested within a lumen of the lead body in a retracted state, and may project beyond the distal end of the lead body in an extended state. A drawback of these known leads is that leak paths may form that permit blood and other organic fluids from the patient to penetrate a central lumen of the lead. For example, the fixation helix must have some clearance within the surrounding components to permit the fixation helix to move relative to the surrounding components. The clearances allow for the formation of leak paths into the central lumen, particularly when the fixation helix is in the extended state and there is an axial gap present within the distal region of the lead. The implantable lead 100 according to embodiments described herein has an integrated blood seal that prevents blood and other organic fluid from penetrating the central lumen. For example, the fixation helix 106 may not translate in distal and proximal directions relative to other parts of the lead (e.g., the header coupling 200 shown in Figure 4).

The terminal connection segment 108 is designed to be received in a receptacle or lead adaptor 56 of the IMD 52 (shown in Figure 1), such a pulse generator device. The terminal connection segment 108 may have an IS1/DF1 configuration, IS4/DF4 configuration, or another configuration. The terminal connection segment 108 may include electrically conductive contacts 122 and electrically insulating portions 124 that alternate with the electrically conductive contacts 122 in a row. The electrically conductive contacts 122 may be ring contacts, pin contacts, and/or the like.

The implantable lead 100 includes multiple electrodes for delivering electrical stimulation to the cardiac tissue of the patient. For example, the implantable lead 100 may include a defibrillation (e.g., shock) coil 116, a tip electrode 118, and one or more ring electrodes 120. The electrodes 116, 118, 120 may be spaced apart along the length of the implantable lead 100. In an embodiment, the fixation helix 106 may be (e.g., represent) the tip electrode 118. The ring electrode(s) 120 may be disposed between the defibrillation coil 116 and the tip electrode 118. The implantable lead 100 has two ring electrodes 120 in the illustrated example, but may have only one or more than two ring electrodes 120 in other example embodiments. In an embodiment, the implantable lead 100 comprises at least one ring electrode 120, the tip electrode 118, and the defibrillation coil 116. In another embodiment, the implantable lead 100 comprises the tip electrode 118 and at least one ring electrode 120, but no defibrillation coil 116.

The implantable lead 100 includes electrical conductors that extend through the lead body 110 from the electrodes 116, 118, 120 to corresponding electrically conductive contacts 122 of the terminal connection segment 108. The electrical conductors may be helical coils, multi-filar conductors, and/or the like. Pacing pulses from the IMD 52 may be conveyed through one or more of the electrical conductors to at least one of the ring and tip electrodes 118, 120 to pace the cardiac tissue. Defibrillation shocks from the IMD 52 may be conveyed through one or more of the electrical conductors to the defibrillation coil 116 to shock the cardiac tissue.

Figure 4 is an elevation view of a header coupling 200 of an implantable lead according to an embodiment. The header coupling 200 may be part of the implantable lead 100 shown in Figure 3. For example, the header coupling 200 may be located in the distal end segment 130 of the implantable lead 100. The header coupling 200 in an embodiment has a unitary, one-piece (e.g., monolithic) body 202. The body 202 continuously extends from a proximal end 204 of the header coupling 200 to a distal end 206 of the header coupling 200. The header coupling 200 has a tubular shape and defines a central lumen 208 therethrough. The central lumen 208 may extend from the proximal end 204 to the distal end 206, and may be open at both ends 204, 206. The central lumen 208 is designed to accommodate at least a portion of a helix shaft, which is inserted into the central lumen 208 through a distal opening 210 of the header coupling 200 (e.g., at the distal end 206).

Figure 5 is a cross-sectional view of the header coupling 200 of Figure 4. The cross-section is taken along line 5-5 in Figure 4 and bisects the header coupling 200. In various embodiments, the header coupling 200 is assembled to a helix shaft to form a sealed joint that seals a central lumen of the implantable lead 100 from blood and other organic fluids. The helix shaft may be mounted to a fixation helix, such as the fixation helix 106 shown in Figure 3.

Referring to both Figures 4 and 5, the header coupling 200 in an embodiment includes a base section 212, a distal tube section 214, and a proximal tube section 216. The base section 212 is disposed between the distal tube section 214 and the proximal tube section 216 along the length of the header coupling 200. The distal tube section 214 extends from the base section 212 to the distal end 206. The proximal tube section 216 extends from the base section 212 to the proximal end 204. The central lumen 208 continuously extends through all three sections 212, 214, 216. The central lumen 208 is defined by an inner surface 222 of the header coupling 200. The inner surface 222 may have a cylindrical shape. In an embodiment, the three sections 212, 214, 216 are different parts of the unitary body 202, so the sections 212, 214, 216 are seamlessly connected. In another embodiment, the header coupling 200 may be defined by two or three components that are coupled together during an assembly process, rather than being parts of a unitary body. For example, the distal tube section 214 and/or the proximal tube section 216 may be screwed into the base section 212 via helical threads, may be chemically bonded via an adhesive, and/or the like. In an embodiment, the base section 212 includes a base flange 218. The base flange 218 is an annular flange that has a greater diameter than the distal tube section 214 and the proximal tube section 216.

In an example embodiment, the distal tube section 214 is designed to be crimped onto the helix shaft that extends into the central lumen 208 through the distal opening 210. For example, the distal tube section 214 has an elongated length to accommodate a crimp tool that is applied onto the outer surface 224 of the header coupling 200 along the distal tube section 214. The crimp tool may be a circumferential crimp tool that surrounds the distal tube section 214 and squeezes the distal tube section 214 radially inward to compress the distal tube section 214 into permanent (e.g., sustained) mechanical contact with the portion of the helix shaft located within the central lumen 208. In an example, the length of the distal tube section 214 may be at least 30% of the axial length of the header coupling 200 (from the proximal end 204 to the distal end 206). In the illustrated example, the length of the distal tube section 214 is at least half of the length of the header coupling 200.

In an embodiment, the distal tube section 214 includes an annular crimp landing area 220. A wall thickness of the distal tube section 214 along the annular crimp landing area 220 is less than the wall thickness of the distal tube section 214 outside of the annular crimp landing area 220. For example, the distal tube section 214 is defined by a cylindrical wall 226. The cylindrical wall 226 is thinner along the annular crimp landing area 220 than along an area of the distal tube section 214 proximal to and/or distal to the annular crimp landing area 220. The annular crimp landing area 200 extends axially from a proximal shoulder 230 of the distal tube section 214 to a distal shoulder 232 of the distal tube section 214. The reduced wall thickness makes the distal tube section 214 more pliable, allowing the distal tube section 214 to be deformed by the crimp tool using less force than if the wall thickness was greater. In an example embodiment, the distal tube section 214 includes a distal radial lip 228 that extends from the distal shoulder 232 to the distal end 206. The distal radial lip 228 has a greater wall thickness than the annular crimp landing area 220. The header coupling 200 in Figures 4 and 5 is shown before the header coupling 200 undergoes the crimping operation. For example, the header coupling 200 in Figures 4 and 5 is in a pre-crimped state. In an example, the header coupling 200 is composed of a metal material. The header coupling 200 may be formed by a molding process or the like.

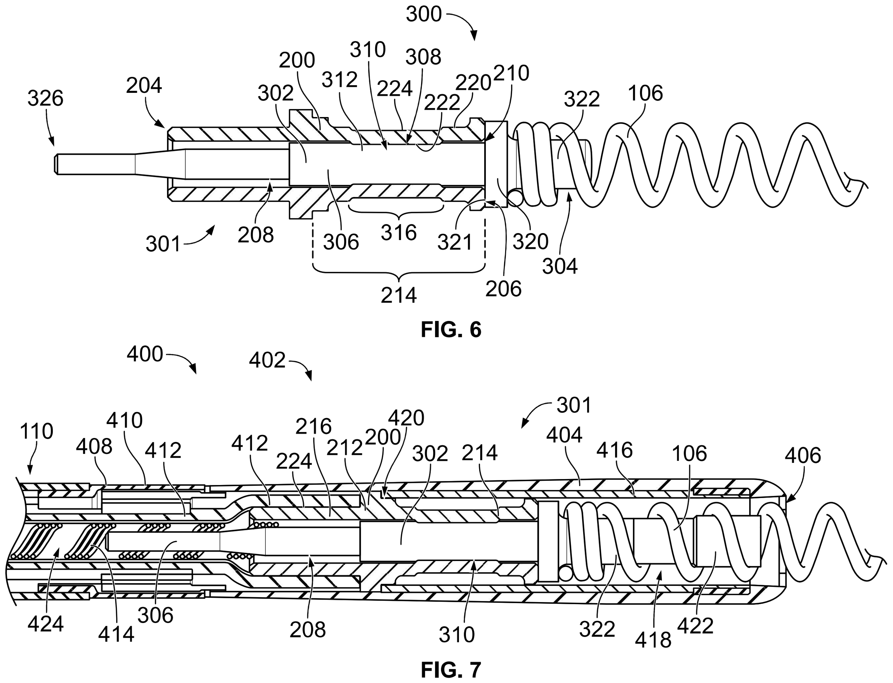

Figure 6 is a partial cross-sectional view of a header assembly 300 of an implantable lead according to an embodiment. The header assembly 300 includes the header coupling 200 shown in Figures 4 and 5. The header assembly 300 in Figure 6 includes the header coupling 200, a helix shaft 302, and the fixation helix 106. The header coupling 200 is shown in cross-section to enable viewing the inside of the central lumen 208. The other components are not shown in cross-section. The header assembly 300 may be part of the implantable lead 100 shown in Figure 3. For example, the header assembly 300 may be located in the distal end segment 130 of the implantable lead 100.

The fixation helix 106 is mounted to a distal segment 304 of the helix shaft 302. The fixation helix 106 is configured to penetrate tissue of a patient, as described above. The helix shaft 302 also includes a proximal shank 306 that extends into the central lumen 208 of the header coupling 200. The proximal shank 306 enters the central lumen 208 through the distal opening 210 of the distal tube section 214. When assembled, a surface of the header coupling 200 directly engages and is fixedly secured to a surface of the helix shaft 302, forming a sealed joint 308 that seals the central lumen 208 of the header coupling 200 and prevents fluid from migrating past the distal end 206 and into a central lumen of the lead 100.

In the illustrated embodiment, the sealed joint 308 is a crimp joint 310. The crimp joint 310 is located along the distal tube section 214 of the header coupling 200. For example, even if blood or other organic fluid can access the distal opening 210 of the header coupling 200, the crimp joint 310 blocks the blood or other organic fluid from moving in a proximal direction beyond the crimp joint 310. In the illustrated embodiment, the crimp joint 310 is defined between the inner surface 222 of the header coupling 200 (along the distal tube section 214) and an outer surface 312 of the proximal shank 306. The crimp joint 310 is formed by applying a crimp tool (e.g., a crimping die) onto the distal tube section 214 and then applying radial force via the crimp tool to squeeze the header coupling 200 against the proximal shank 306 of the helix shaft 302. The terms "crimp" and "crimping" as used herein encompass swaging. The crimp joint 310 is a mechanical seal. In an example, both the helix shaft 302 and the header coupling 200 are composed of metal. The crimp joint 310 may be a metal-to-metal mechanical joint.

The crimping operation deforms the distal tube section 214 and the proximal shank 306. For example, the interface between the inner surface 222 and the outer surface 312 may be irregular or otherwise altered in the area of the crimp joint 310, relative to the interface outside of the area of the crimp joint 310. The crimp joint 310 aligns with the annular crimp landing area 220 of the distal tube section 214. For example, the crimp tool may be applied on the outer surface 224 of the distal tube section 214 along the annular crimp landing area 220. Due to the force exerted by the crimp tool on the outer surface 224, the distal tube section 214 may have an annular imprint 316 along the outer surface 224 after the crimping operation. The annular imprint 316 may be a groove or other indentation caused by the compressive force of the crimp tool. In an embodiment, the annular imprint 316 is located along the annular crimp landing area 220. The crimp joint 310 locks the helix shaft 302 to the header coupling 200. For example, after the crimping operation, the helix shaft 302 is axially and rotationally fixed in place relative to the header coupling 200. The helix shaft 302 may be inseparable from the header coupling 200 due to the crimp joint 310.

In the illustrated embodiment, the helix shaft 302 includes an intermediate flange 320 disposed between the proximal shank 306 and the distal segment 304 of the helix shaft 302. The distal end 206 of the header coupling 200 may abut a proximal face 321 of the intermediate flange 320. For example, during assembly, the proximal shank 306 may be loaded into the central lumen 208 of the header coupling 200 until the proximal face 321 of the intermediate flange 320 abuts against the distal end 206 of the header coupling 200, blocking further advancement into the central lumen 208. The crimping operation may be performed when the distal end 206 is in contact with the proximal face 321. Optionally, the distal end 206 may be at least slightly spaced apart from the proximal face 321 after the crimping operation. For example, the crimp joint 310 is proximal to the distal end 206 of the header coupling 200, so there may be no need to ensure that the distal end 206 remains in contact with the intermediate flange 320 of the helix shaft 302 once the crimp joint 310 is established.

The fixation helix 106 is secured to the distal segment 304 of the helix shaft 302. In the illustrated embodiment, the distal segment 304 of the helix shaft 302 includes a distal boss 322. For example, the intermediate flange 320 may be axially disposed between the proximal shank 306 and the distal boss 322. The distal boss 322 may extend from the intermediate flange 320 to a distal end of the helix shaft 302. A proximal segment of the fixation helix 106 may surround and mechanically engage a perimeter surface of the distal boss 322 to mount the fixation helix 106 to the helix shaft 302. Optionally, the mount may be reinforced by welding the fixation helix 106 to the distal boss 322, by applying an adhesive that bonds the fixation helix 106 to the distal boss 322, and/or the like. In another optional example, the distal boss 322 may include one or more protrusions, such as tabs or helical threads, which mechanically couple to the proximal segment of the fixation helix 106 to reinforce the connection. In another embodiment, the helix shaft 302 may lack the distal boss 322 shown in Figure 6. For example, the proximal end of the fixation helix 106 may be welded, bonded, or otherwise secured to a distal face of the intermediate flange 320 of the helix shaft 302.

In an embodiment, the fixation helix 106 is axially fixed in place relative to the header coupling 200 via the crimp joint 310 and the mounting of the fixation helix 106 to the helix shaft 302. For example, the header coupling 200, the helix shaft 302, and the fixation helix 106 may be secured together as a unit. In an example, the fixation helix 106 is rotationally fixed to the header coupling 200 via the helix shaft 302. As a result, the three components 106, 200, 302 of the header assembly 300, i.e., the fixation helix 106, the header coupling 200, and the helix shaft 302, may rotate together, without relative rotational movement. As such, torque applied to the proximal end of the helix shaft 302 and/or to the proximal end 204 of the header coupling 200 in a first rotational direction, such as clockwise, may cause the header assembly 300 to rotate in the first rotational direction relative to the patient. The torque may be applied by a stylet (e.g., a locking stylet) that is inserted through a central lumen of the implantable lead 100. The rotation in the first direction may cause the fixation helix 106 to worm in an advancing direction into the patient tissue. Torque applied in an opposite, second rotational direction, such as counterclockwise, may cause the header assembly 300 to rotate together in the second rotational direction, which causes the fixation helix to worm in a retreating direction out of the patient tissue.

Figure 7 is a partial cross-sectional view of a distal end segment 402 of an implantable lead 400 according to an embodiment. The distal end segment 402 includes the header assembly 300 shown in Figure 6. The distal end segment 402 may be the distal end segment 130 of the implantable lead 100 shown in Figure 3. For example, the implantable lead 400 may be the implantable lead 100 shown in Figure 3. Figure 7 represents an example lead configuration that includes the header assembly 300. The header assembly 300 can be part of different implantable leads in other embodiments.

The distal end segment 402 includes an outer jacket 404 that surrounds the header coupling 200, the helix shaft 302, and a portion of the fixation helix 106. A distal portion of the fixation helix 106 projects beyond a distal end 406 of the outer jacket 404. The outer jacket 404 may be an electrically insulative and tubular member, such as a sleeve. In an example, the outer jacket 404 is composed of silicone. In another example, the outer jacket 404 may be composed of a different polymer material. The outer jacket 404 in an embodiment may extend from a ring electrode 408 of the implantable lead 400 to the distal end 406. For example, the outer jacket 404 may extend from an edge of an exposed portion 410 of the ring electrode 408. The exposed portion 410 may be exposed to the organic tissue and fluids of the patient, and may function as an electrode surface for delivering pacing pulses and/or sensing electrical signals.

In an embodiment, the implantable lead 400 includes an inner jacket 412 and an inner coil 414 within the inner jacket 412. The inner jacket 412 may surround and mechanically engage the outer surface 224 of the header coupling 200 along the proximal tube section 216. The inner jacket 412 may be an electrically insulative and tubular member, such as a sleeve. The inner coil 414 may surround and mechanically and electrically engage a portion of the proximal shank 306 of the helix shaft 302. The inner coil 414 is electrically conductive. The inner coil 414 may be composed of a metal material. The inner coil 414 may be an electrical conductor that extends through the lead body 110 to electrically connect an electrode to a corresponding electrically conductive contact 122 of the terminal connection segment 108 (shown in Figure 3). For example, the inner coil 414 may be electrically connected to the fixation helix 106, which functions as a tip electrode. The inner coil 414 may be electrically connected to the fixation helix 106 via the helix shaft 302.

In an embodiment, the distal end segment 402 of the implantable lead 400 includes a header can 416 that has a cylindrical shape and defines a cavity 418. The distal tube section 214 of the header coupling 200 is disposed within the cavity 418. The header can 416 may be composed of a metal material. A proximal end 420 of the header can 416 may be secured to the base section 212 and/or the distal tube section 214 of the header coupling 200. For example, the proximal end 420 may be welded to the base section 212 of the header coupling 200. The outer jacket 404 may surround the header can 416.

In an embodiment, the distal end segment 402 includes a cylindrical support member 422 located distal to the helix shaft 302 and surrounded by the fixation helix 106. For example, the cylindrical surround member 422 may be disposed next to the distal boss 322 of the helix shaft 302. The fixation helix 106 may coil around the cylindrical support member 422 within the cavity 418 of the header can 416 and within the outer jacket 404. The cylindrical support member 422 may be optional. The distal end segment 402 may lack the cylindrical support member 422 in another embodiment.

The inner coil 414 and/or the inner jacket 412 define a central lumen 424. The central lumen 424 may extend at least a majority of the length of the implantable lead 400 from the distal end segment 402 towards a proximal end 420 of the implantable lead 400. The central lumen 424 is referred to herein as a lead central lumen 424. The lead central lumen 424 is fluidly connected to and coaxial with the central lumen 208 of the header coupling 200. The central lumen 424 provides a passageway for tools, such as stylets, to be inserted into the implantable lead 400. The tools can be used to assist with maneuvering the implantable lead 400 through the transvenous system of the patient during implant and explant. The crimp joint 310 seals the central lumen 208 of the header coupling 200, which constructively seals a distal end 406 of the lead central lumen 424 preventing fluid from migrating past the distal end 406 and into a central lumen of the lead 100. For example, the inner coil 414 and/or the inner jacket 412 may surround and seal to the proximal tube section 216 of the header coupling 200. The central lumen 208 of the header coupling 200 functions as an extension of the lead central lumen 424. The crimp joint 310 prevents blood and other organic fluids from entering the lead central lumen 424 through the header coupling 200. Due to the crimp joint 310, the lead central lumen 424 remains free (e.g., devoid) of coagulated blood and other bodily fluids. Locking stylets and other tools can access the distal end segment 402 of the implantable lead 400 through the lead central lumen 424 without being obstructed by blood or other bodily fluids. In all, the crimp joint 310 prevents migrating fluid to fill the central lumen 424, resulting in coagulation, thus blocking off a long portion of central lumen 424 of the implantable lead 400. This results in full access to the central lumen 424, providing a benefit during extraction of the implantable lead 400 at a later time because extraction wires can be fully inserted from the proximal end 420 and anchored at the distal end 406.

In Figure 7, the outer jacket 404 is described as a monolithic tubular member (e.g., sleeve). In another embodiment, the outer jacket may be a combination of multiple components. For example, the outer jacket may be defined by a sleeve and a distal tip member. The distal tip member may extend from a distal end of the sleeve to the distal end 406.

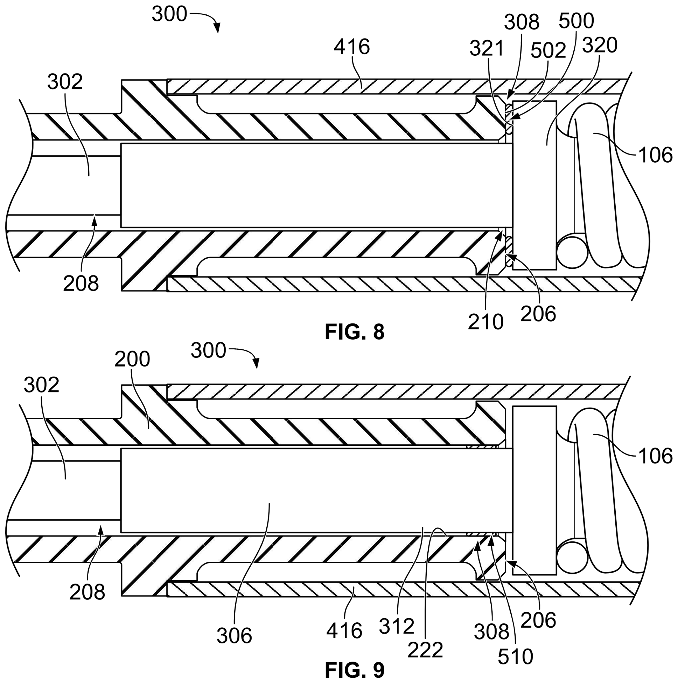

Figure 8 is a close-up, partial cross-sectional view of the header assembly 300 shown in Figures 6 and 7 according to a second embodiment. The header assembly 300 includes the header coupling 200, the helix shaft 302, and the fixation helix 106. The header can 416 is also shown. The header coupling 200 and the header can 416 are shown in cross-section. Similar to the embodiment shown in Figures 6 and 7, the header coupling 200 directly engages and is fixedly secured to the helix shaft 302 via the sealed joint 308. The sealed joint 308 seals the central lumen 208 of the header coupling 200 and prevents fluid from migrating past the distal end 206 and into a central lumen of the lead 100. In the illustrated embodiment, the sealed joint 308 is a weld joint 500. The weld joint 500 is formed between the distal end 206 of the header coupling 200 and the proximal face 321 of the intermediate flange 320 of the helix shaft 302. The weld joint 500 may circumferentially extend along the full annular face of the header coupling 200. The weld joint 500 seals the distal opening 210 of the distal tube section 214. The weld joint 500 blocks blood and/or other organic fluid from entering the central lumen 208 through the distal opening 210. The weld joint 500 may be formed by spot welding the distal end 206 of the header coupling 200 to the intermediate flange 320. The welding process uses high temperature to permanently connect the distal face 502 of the header coupling 200 to the proximal face 321 of the intermediate flange 320 of the helix shaft 302 at the weld joint 500.

Figure 9 is a close-up, partial cross-sectional view of the header assembly 300 according to a third embodiment. The third embodiment shown in Figure 9 is similar to the second embodiment shown in Figure 8 because the sealed joint 308 is a weld joint. The weld joint 510 in Figure 9 is within the central lumen 208 of the header coupling 200. For example, the weld joint 510 is formed between the outer surface 312 of the proximal shank 306 of the helix shaft 302 and the inner surface 222 of the header coupling 200. The weld joint 510 may be at any location along the length of the header coupling 200. In one example, the weld joint 510 is located at or proximate to (e.g., within 1 mm or 2 mm) the distal end 206 of the header coupling 200, as shown in Figure 9. In another example, the weld joint 510 may be spaced farther away from the distal end 206. The weld joint 510 functions the same as the weld joint 500 in Figure 8, sealing the central lumen 208 to prevent blood and other fluids from penetrating through the central lumen 208 (e.g., into the lead central lumen 424 shown in Figure 7). The weld joint 510 may be formed by spot welding the inner surface 222 of the header coupling 200 to the outer surface 312 of the helix shaft 302. The welding process uses high temperature to permanently connect the inner surface 222 to the outer surface 312 at the weld joint 510.

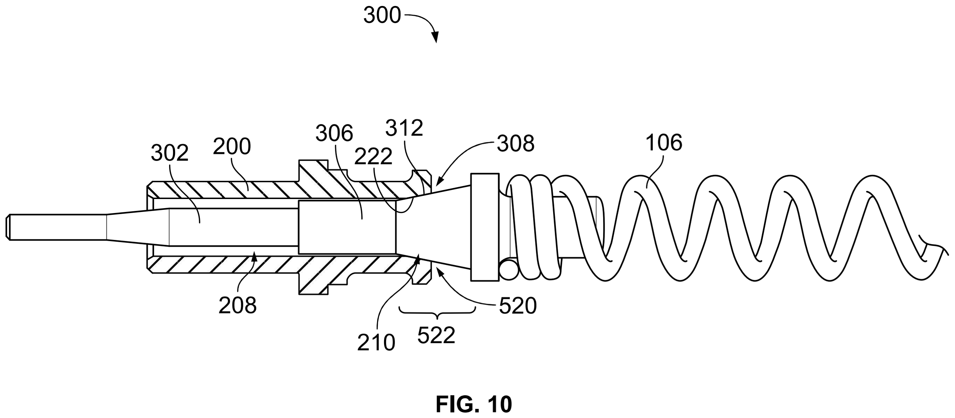

Figure 10 is a close-up, partial cross-sectional view of the header assembly 300 according to a fourth embodiment. The header assembly 300 includes the header coupling 200, the helix shaft 302, and the fixation helix 106. The header coupling 200 is shown in cross-section. Similar to the embodiments shown in Figures 6 through 9, the header coupling 200 directly engages and is fixedly secured to the helix shaft 302 via a sealed joint 308. The sealed joint 308 seals the central lumen 208 of the header coupling 200 and prevents fluid from migrating past the distal end 206 and into a central lumen of the lead 100. In the illustrated embodiment, the sealed joint 308 is defined by a press-fit mating interface 520 between the inner surface 222 of the header coupling 200 and the outer surface 312 of the helix shaft 302. The proximal shank 306 of the helix shaft 302 tapers in a proximal direction along at least a mating section 522 of the proximal shank 306. For example, a proximal end of the mating section 522 of the proximal shank 306 has a smaller diameter than a distal end of the mating section 522. The mating section 522 forms the press-fit mating interface 520 with the inner surface 222 of the header coupling 200 when the proximal shank 306 is inserted into the central lumen 208 through the distal opening 210. The press-fit mating interface 520 may be defined by the direct surface-to-surface contact of the two components. The helix shaft 302 may be loaded into the central lumen 208 through the distal opening 210 with sufficient mechanical force to reliably seal the central lumen 208 against penetration by blood and other bodily fluids.

In various alternative embodiments, the header coupling 200 may lack the proximal tube section 216. For example, the header coupling 200 may only include the base section 212 and the distal tube section 214. The base section 212 may define the proximal end of the header coupling 200. The inner coil 414 and/or the inner jacket 412 of the implantable lead 400 (shown in Figure 7) may be secured and sealed directly to the base section 212 of the header coupling 200. This first alternative header coupling design could replace the header coupling 200 shown in Figures 4 through 10.

In a second alternative header coupling design, the header coupling 200 may lack the distal tube section 214. For example, the header coupling 200 may only include the proximal tube section 216 and the base section 212. The base section 212 may define a distal opening to the central lumen 208. In a first alternative embodiment, the sealed joint 308 is defined by a press-fit mating interface between the inner surface 222 of the header coupling 200, at the distal end of the base section 212, and the outer surface 312 of the helix shaft 302. For example, the tapered mating section 522 shown in Figure 10 may form a press-fit mating interface with the inner surface 222 along the base section 212 of the header coupling 200. In a second alternative embodiment using the header coupling that lacks the distal tube section, the sealed joint 308 is a weld joint similar to the weld joints shown in Figures 8 and 9. The weld joint may be between the outer surface 312 of the proximal shank 306 of the helix shaft 302 and the inner surface 222 of the header coupling, along either the base section 212 or the proximal tube section 216. In a third alternative embodiment using the header coupling that lacks the distal tube section, the sealed joint 308 is a crimp joint that is located along the proximal tube section 216 of the header coupling 200. For example, a crimp tool may radially compress the proximal tube section 216 into permanent engagement with the proximal shank 306 of the helix shaft 302 extending through the proximal tube section 216. In general, the various features and examples described herein can be combined unless the combination of a first feature with a second feature would frustrate the function of one of the features or render one of the features useless.

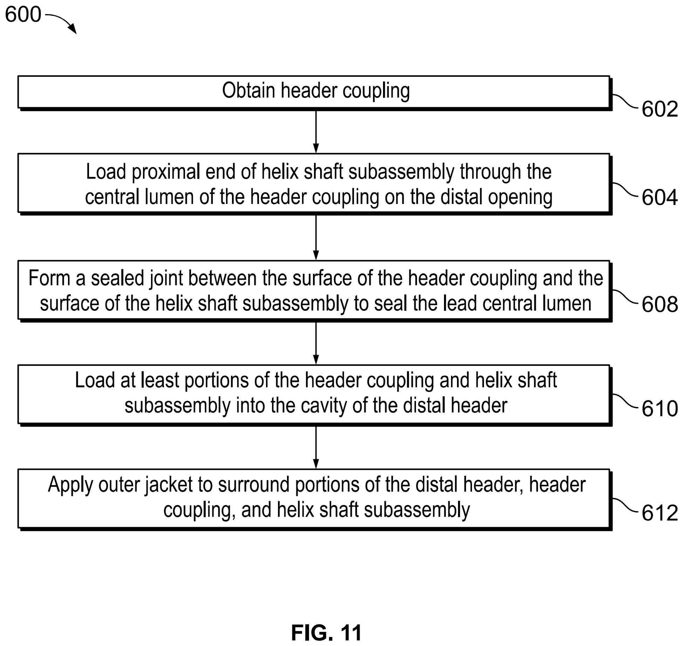

Figure 11 is a flow chart of a method 600 for assembling a distal end segment of an implantable lead according to an embodiment. The method 600 may be used to assemble the distal end segment 300, and components thereof, shown in Figures 4 through 10. In different embodiments, the method may include different steps not shown in Figure 11, may omit one or more of the steps shown in Figure 11, and/or may have a different order of the steps than shown in Figure 11. The order of the steps may vary from the order that is presented below. In one example, step 606 may occur prior to step 604.

At step 602, a header coupling 200 is obtained that extends from a proximal end 204 thereof to a distal end 206 thereof. The header coupling 200 may have a tubular shape. The header coupling 200 defines a central lumen 208 that extends from the proximal end 204 to the distal end 206. The header coupling 200 including a base section 212 and a distal tube section 214 extending from the base section 212 to the distal end 206. The header coupling 200 may also include a proximal tube section 216 extending from the base section 212 to the proximal end 204.

At step 604, a proximal shank 306 of a helix shaft 302 is loaded into the central lumen 208 of the header coupling 200 through a distal opening 210 of the distal tube section 214.

At step 606, a fixation helix 106 is mounted to a distal segment 304 of the helix shaft 302. The fixation helix 106 is configured to penetrate tissue of a patient. In an example, the distal segment 304 of the helix shaft 302 includes a distal boss 322. Mounting the fixation helix 106 to the distal segment 304 of the helix shaft 302 may include loading the fixation helix 106 on the distal boss 322 to surround and engage a perimeter surface of the distal boss 322.

At step 608, a sealed joint 308 is formed between a surface of the header coupling 200 and a surface of the helix shaft 302 to seal the central lumen 208 of the header coupling 200. The surface of the header coupling 200 directly engages and is fixedly secured to the surface of the helix shaft 302 at the sealed joint 308.

In a first embodiment, forming the sealed joint 308 includes crimping the distal tube section 214 of the header coupling 200 onto the proximal shank 306 of the helix shaft 302, forming a crimp joint 310 that seals the central lumen 208 of the header coupling 200 and prevents fluid from migrating past the distal end 206 and into a central lumen of the lead 100. The distal tube section 214 may include an annular crimp landing area 220. A wall thickness of the distal tube section 214 along the annular crimp landing area 220 is less than the wall thickness of the distal tube section 214 outside of the annular crimp landing area 220. The crimping may cause the crimp joint 310 to be located along the annular crimp landing area 220.

In a second embodiment, forming the sealed joint 308 includes forming a weld joint 500, 510 by welding the surface of the header coupling 200 to the surface of the helix shaft 302. The helix shaft 302 may include an intermediate flange 320 disposed between the proximal shank 306 and the distal segment 304 of the helix shaft 302. The weld joint 500 may be formed by welding the distal end 206 of the header coupling 200 to a proximal face 321 of the intermediate flange 320 of the helix shaft 302. The weld joint 510 may be formed by welding an inner surface 222 the header coupling 200 to an outer surface 312 of the proximal shank 306 of the helix shaft 302.

In a third embodiment, forming the sealed joint 308 includes press-fit mating an outer surface 312 of the helix shaft 302 against an inner surface 222 of the header coupling 200 that defines the central lumen 208. In an example, the proximal shank 306 of the helix shaft 302 tapers in a proximal direction along at least a mating section 522 of the proximal shank 302. The press-fit mating includes inserting the proximal shank 302 into the central lumen 208 through the distal opening 210 of the distal tube section 214 to form a press-fit mating interface 520 with the inner surface 222 of the header coupling 200.

At step 610, the distal tube section 214 of the header coupling 200, at least a portion of the helix shaft 302, and a portion of the fixation helix 106 are loaded into a cavity 418 of a header can 416. The header can 416 may be composed of a metal material. The header can 416 may have a cylindrical shape. The method may include securing a proximal end 420 of the header can 416 to the base section 212 of the header coupling 200.

At step 612, an outer jacket 404 is applied to surround at least the header coupling 200, the helix shaft 302, and a first portion of the fixation helix 106. A second portion of the fixation helix 106 may project beyond a distal end 406 of the outer jacket 404. The outer jacket 404 also surrounds the header can 416.

The method 600 may include one or more additional steps. The additional step(s) may involve coupling the assembled distal end segment to other components of the implantable lead, such as an inner coil, an inner jacket, a ring electrode, and/or the like.

An aspect of invention relates to an implantable lead 400 comprising a header coupling 200 extending from a proximal end 204 of the header coupling 200 to a distal end 206 of the header coupling 200. The header coupling 200 defining a central lumen 208 that extends from the proximal end 204 to the distal end 206. The header coupling 200 including a base section 212 and a distal tube section 214 extending from the base section 212 to the distal end 206. The implantable lead 400 also comprises a helix shaft 302 comprising a proximal shank 306 that extends into the central lumen 208 of the header coupling 200 through a distal opening 210 of the distal tube section 214. The implantable lead 400 further comprises a fixation helix 106 mounted to a distal segment 304 of the helix shaft 302 and configured to penetrate tissue of a patient. A surface 222 of the header coupling 200 directly engages and is fixedly secured to a surface 312 of the helix shaft 302, forming a sealed joint 308 that prevents fluid from migrating past the distal end 206.

In an embodiment, the sealed joint 308 is a crimp joint 310 and is located along the distal tube section 214 of the header coupling 200. The surface 222 of the header coupling 200 that forms the sealed joint 308 is an inner surface 222 that defines the central lumen 208, and the surface 312 of the helix shaft 302 that forms the sealed joint 308 is an outer surface 312 of the proximal shank 306.

In an embodiment, the distal tube section 214 has an annular imprint 316 along an outer surface 224 of the header coupling 200. The annular imprint 316 attributable to a crimping tool.

In an embodiment, the distal tube section 214 includes an annular crimp landing area 220. A wall thickness of the distal tube section 214 along the annular crimp landing area 220 is less than the wall thickness of the distal tube section 214 outside of the annular crimp landing area 220.

In an embodiment, the implantable lead 400 further comprises an outer jacket 404 that surrounds the header coupling 200 and the helix shaft 302. A portion of the fixation helix 106 projects beyond a distal end 406 of the outer jacket 404.

In an embodiment, the fixation helix 106 is axially fixed in place relative to the header coupling 200 via the sealed joint 308 between the header coupling 200 and the helix shaft 302.

In an embodiment, the sealed joint 308 is a weld joint 500, 510.

In an embodiment, the helix shaft 302 includes an intermediate flange 320 disposed between the proximal shank 306 and the distal segment 304 of the helix shaft 302, and the distal end 206 of the header coupling 200 abuts a proximal face 321 of the intermediate flange 320 of the helix shaft 302.

In an embodiment, the sealed joint 308 is a weld joint 500 between the distal end 206 of the header coupling 200 and the proximal face 321 of the intermediate flange 320 of the helix shaft 302.

In an embodiment, the sealed joint 308 is defined by a press-fit mating interface 520 between an inner surface 222 of the header coupling 200 that defines the central lumen 208 and an outer surface 312 of the helix shaft 302.

In an embodiment, the proximal shank 306 of the helix shaft 302 tapers in a proximal direction along at least a mating section 522 of the proximal shank 306. The mating section 522 forming the press-fit mating interface 520 with the inner surface 222 of the header coupling 200 when the proximal shank 306 is inserted into the central lumen 208 through the distal opening 210.

In an embodiment, the header coupling 200 has a proximal tube section 216 extending from the base section 212 to the proximal end 204 of the header coupling 200. In this embodiment, the implantable lead 400 comprises an inner jacket 412 that surrounds and engages an outer surface 224 of the proximal tube section 216. The implantable lead 400 also comprises, in this embodiment, an inner coil 414 within the inner jacket 412. The inner coil 414 surrounding and engaging a portion of the proximal shank 306 of the helix shaft 302.

In an embodiment, the implantable lead 400 further comprising a header can 416 that has a cylindrical shape and defines a cavity 418. A proximal end 420 of the header can 416 is secured to the base section 212 of the header coupling 200, and the distal tube section 214 of the header coupling 200 is disposed within the cavity 418 of the header can 418. In an embodiment, the header can 416 composed of a metal material.

In an embodiment, the distal segment 304 of the helix shaft 302 includes a distal boss 322, and the fixation helix 106 surrounds and engages a perimeter surface of the distal boss 322 to mount the fixation helix 106 to the distal segment 304 of the helix shaft 302.

Another aspect of the invention relates to an implantable lead 400 comprising a header coupling 200 extending from a proximal end 204 of the header coupling 200 to a distal end 206 of the header coupling 200. The header coupling 200 defining a central lumen 208 that extends from the proximal end 204 to the distal end 206. The header coupling 200 including a base section 212 and a distal tube section 214 extending from the base section 212 to the distal end 206. The implantable lead 400 also comprises a helix shaft 302 comprising a proximal shank 306 that extends into the central lumen 208 of the header coupling 200 through a distal opening 210 of the distal tube section 214. The implantable lead 400 further comprises a fixation helix 106 mounted to a distal segment 304 of the helix shaft 302 and configured to penetrate tissue of a patient. The distal tube section 214 of the header coupling 200 is crimped onto the proximal shank 306 of the helix shaft 302, forming a crimp joint 310 that seals the central lumen 208 of the header coupling 200.

In an embodiment, an inner surface 222 of the header coupling 200 that defines the central lumen 208 directly engages and is fixedly secured to an outer surface 312 of the proximal shank 306 via the crimp joint 310.

In an embodiment, the distal tube section 214 has an annular imprint 316 along an outer surface 224 of the header coupling 200. The annular imprint 316 attributable to a crimping tool.

A further aspect of the invention relates to a method for assembling a distal end segment 140 of an implantable lead 400. The method comprising obtaining a header coupling 200 that extends from a proximal end 204 of the header coupling 200 to a distal end 206 of the header coupling 200. The header coupling 200 defining a central lumen 208 that extends from the proximal end 204 to the distal end 206. The header coupling 200 including a base section 212 and a distal tube section 214 extending from the base section 212 to the distal end 206. The method also comprises loading a proximal shank 306 of a helix shaft 302 into the central lumen 208 of the header coupling 200 through a distal opening 210 of the distal tube section 214. The method further comprises mounting a fixation helix 106 to a distal segment 304 of the helix shaft 302. The fixation helix 106 configured to penetrate tissue of a patient. The method additionally comprises forming a sealed joint 308 between a surface 222 of the header coupling 202 and a surface 312 of the helix shaft 302 to seal the central lumen 208 of the header coupling 200 and to prevent fluid from migrating past the distal end 206. The surface 222 of the header coupling 200 directly engages and is fixedly secured to the surface 312 of the helix shaft 302 at the sealed joint 308.

In an embodiment, forming the sealed joint 308 comprises crimping the distal tube section 214 of the header coupling 200 onto the proximal shank 306 of the helix shaft 302, forming a crimp joint 310 that seals the central lumen 208 of the header coupling 200.

In an embodiment, the distal tube section 214 includes an annular crimp landing area 200. In this embodiment, a wall thickness of the distal tube section 214 along the annular crimp landing area 220 is less than the wall thickness of the distal tube section 214 outside of the annular crimp landing area 200. In this embodiment, the crimping causes the crimp joint 310 to be located along the annular crimp landing area 200.

In an embodiment, forming the sealed joint 308 comprises forming a weld joint 500, 510 by welding the surface 222 of the header coupling 200 to the surface 312 of the helix shaft 302.

In an embodiment, the helix shaft 302 includes an intermediate flange 320 disposed between the proximal shank 306 and the distal segment 304 of the helix shaft 302, and forming the weld joint 500, 510 comprises welding the distal end 206 of the header coupling 200 to a proximal face 321 of the intermediate flange 320 of the helix shaft 302.

In an embodiment, forming the sealed joint 308 comprises press-fit mating an outer surface 312 of the helix shaft 302 against an inner surface 222 of the header coupling 200 that defines the central lumen 208.

In an embodiment, the proximal shank 306 of the helix shaft 302 tapers in a proximal direction along at least a mating section 522 of the proximal shank 306, and the press-fit mating comprises inserting the proximal shank 306 into the central lumen 208 through the distal opening 210 of the distal tube section 214 to form a press-fit mating interface 520 with the inner surface 222 of the header coupling 200.

In an embodiment, the method further comprising loading the distal tube section 214 of the header coupling 200, at least a portion of the helix shaft 302, and a portion of the fixation helix 106 into a cavity 418 of a header can 416. In an embodiment, the header can 416 composed of a metal material and having a cylindrical shape. The method also comprises, in this embodiment, securing a proximal end 420 of the header can 416 to the base section 212 of the header coupling 200.

In an embodiment, the method further comprising applying an outer jacket 404 to surround the header coupling 200 and the helix shaft 302. A portion of the fixation helix 106 projects beyond a distal end 406 of the outer jacket 404.

In an embodiment, the distal segment 304 of the helix shaft 302 includes a distal boss 322, and mounting the fixation helix 106 to the distal segment 304 of the helix shaft 302 comprises loading the fixation helix 106 on the distal boss 322 to surround and engage a perimeter surface of the distal boss 322.

Yet another aspect of the invention relates to an implantable lead 400 comprising a header coupling 200 extending from a proximal end 204 of the header coupling 200 to a distal end 206 of the header coupling 200. The header coupling 200 defining a central lumen 208 that extends from the proximal end 204 to the distal end 206. The implantable lead 400 also comprises a helix shaft 302 comprising a proximal shank 306 that extends into the central lumen 208 of the header coupling 200. The implantable lead 400 further comprises a fixation helix 106 mounted to a distal segment 304 of the helix shaft 302 and configured to penetrate tissue of a patient. A surface 222 of the header coupling 200 directly engages and is fixedly secured to a surface 312 of the helix shaft 302, forming a sealed joint 308 that seals the central lumen 208 of the header coupling 200 and to prevent fluid from migrating past the distal end 206. The sealed joint 308 is one of (i) a press-fit mating interface 520 between an inner surface 222 of the header coupling 200 that defines the central lumen 208 and an outer surface 312 of the helix shaft 302, or (ii) a weld joint 500, 510 between the surface 222 of the header coupling 200 and the surface 312 of the helix shaft 302.

In an embodiment, the sealed joint 308 is the weld joint 500, 510, and the weld joint 500, 510 is defined between a distal end surface 222 of the header coupling 200 and the outer surface 312 of the helix shaft 302.

In an embodiment, the sealed joint 308 is the press-fit mating interface 520, and the proximal shank 306 of the helix shaft 302 tapers in a proximal direction along at least a mating section 522 of the proximal shank 306 that forms the press-fit mating interface 520 with the inner surface 222 of the header coupling 200 when the proximal shank 306 is inserted into the central lumen 208 through the distal opening 210 of the distal tube section 214.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

The term "sized" as used herein is not limited to the act of manufacturing, but rather refers to a dimension similar to length, width, volume, etc. A lumen being sized to accommodate a specific component is not a method operation, but rather a characteristic of the lumen. The term "about" or "approximately" immediately preceding a stated numerical value, as used herein, indicates that the actual value can be +/- a designated threshold of the stated numerical value. The designated threshold may be 5%, 10% or the like of the stated numerical value.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the inventive subject matter without departing from its scope. While the dimensions and types of materials described herein are intended to define the parameters of the inventive subject matter, they are by no means limiting and are exemplary embodiments. Many other embodiments will be apparent to one of ordinary skill in the art upon reviewing the above description. The scope of the inventive subject matter should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An implantable lead (400) comprising:
a header coupling (200) extending from a proximal end (204) of the header coupling (200) to a distal end (206) of the header coupling (200), the header coupling (200) defining a central lumen (208) that extends from the proximal end (204) to the distal end (206), the header coupling (200) including a base section (212) and a distal tube section (214) extending from the base section (212) to the distal end (206);
a helix shaft (302) comprising a proximal shank (306) that extends into the central lumen (208) of the header coupling (200) through a distal opening (210) of the distal tube section (214); and
a fixation helix (106) mounted to a distal segment (304) of the helix shaft (302) and configured to penetrate tissue of a patient,
wherein a surface (222) of the header coupling (200) directly engages and is fixedly secured to a surface (312) of the helix shaft (302), forming a sealed joint (308) that prevents fluid from migrating past the distal end (206).

2. The implantable lead of claim 1, wherein the sealed joint (308) is a crimp joint (310) and is located along the distal tube section (214) of the header coupling (200), wherein the surface (222) of the header coupling (200) that forms the sealed joint (308) is an inner surface (222) that defines the central lumen (208), and the surface (312) of the helix shaft (302) that forms the sealed joint (308) is an outer surface (312) of the proximal shank (306).

3. The implantable lead of claim 2, wherein the distal tube section (214) has an annular imprint (316) along an outer surface (224) of the header coupling (200), the annular imprint (316) attributable to a crimping tool.

4. The implantable lead of claim 2 or 3, wherein the distal tube section (214) includes an annular crimp landing area (220), wherein a wall thickness of the distal tube section (214) along the annular crimp landing area (220) is less than the wall thickness of the distal tube section outside (214) of the annular crimp landing area (220).

5. The implantable lead of any one of claims 1 to 4, further comprising an outer jacket (404) that surrounds the header coupling (200) and the helix shaft (302), wherein a portion of the fixation helix (106) projects beyond a distal end (406) of the outer jacket (404).

6. The implantable lead of any one of claims 1 to 5, wherein the fixation helix (106) is axially fixed in place relative to the header coupling (200) via the sealed joint (308) between the header coupling (200) and the helix shaft (302).

7. The implantable lead of claim 1, wherein the sealed joint (308) is a weld joint (500, 510).

8. The implantable lead of claim 1 or 7, wherein the helix shaft (302) includes an intermediate flange (320) disposed between the proximal shank (306) and the distal segment (304) of the helix shaft (302), and the distal end (206) of the header coupling (200) abuts a proximal face (321) of the intermediate flange (320) of the helix shaft (302).

9. The implantable lead of claim 8, wherein the sealed joint (308) is a weld joint (500) between the distal end (206) of the header coupling (200) and the proximal face (321) of the intermediate flange (320) of the helix shaft (302).

10. The implantable lead of claim 1, wherein the sealed joint (308) is defined by a press-fit mating interface (520) between an inner surface (222) of the header coupling (200) that defines the central lumen (208) and an outer surface (312) of the helix shaft (302).

11. The implantable lead of claim 10, wherein the proximal shank (306) of the helix shaft (302) tapers in a proximal direction along at least a mating section (522) of the proximal shank (306), the mating section (522) forming the press-fit mating interface (520) with the inner surface (222) of the header coupling (200) when the proximal shank (306) is inserted into the central lumen (208) through the distal opening (201).

12. The implantable lead of any one of claims 1 to 11, wherein the header coupling (200) has a proximal tube section (216) extending from the base section (212) to the proximal end (204) of the header coupling (200), wherein the implantable lead (400) comprises:
an inner jacket (412) that surrounds and engages an outer surface (224) of the proximal tube section (216); and
an inner coil (414) within the inner jacket (412), the inner coil (414) surrounding and engaging a portion of the proximal shank (306) of the helix shaft (302).

13. The implantable lead of any one of claims 1 to 12, further comprising a header can (416) that has a cylindrical shape and defines a cavity (418), wherein a proximal end (420) of the header can (416) is secured to the base section (212) of the header coupling (200), and the distal tube section (214) of the header coupling (200) is disposed within the cavity (418) of the header can (416), the header can (416) composed of a metal material.

14. The implantable lead of any one of claims 1 to 13, wherein the distal segment (304) of the helix shaft (302) includes a distal boss (322), and the fixation helix (106) surrounds and engages a perimeter surface of the distal boss (322) to mount the fixation helix (106) to the distal segment (304) of the helix shaft (302).

15. A method for assembling a distal end segment (140) of an implantable lead (400), the method comprising:
obtaining a header coupling (200) that extends from a proximal end (204) of the header coupling (200) to a distal end (206) of the header coupling (200), the header coupling (200) defining a central lumen (208) that extends from the proximal end (204) to the distal end (206), the header coupling (200) including a base section (212) and a distal tube section (214) extending from the base section (212) to the distal end (206);
loading a proximal shank (306) of a helix shaft (302) into the central lumen (208) of the header coupling (200) through a distal opening (210) of the distal tube section (214);
mounting a fixation helix (106) to a distal segment (304) of the helix shaft (302), the fixation helix (106) configured to penetrate tissue of a patient; and
forming a sealed joint (308) between a surface (22) of the header coupling (200) and a surface (312) of the helix shaft (302) to seal the central lumen (208) of the header coupling (200) and to prevent fluid from migrating past the distal end (206), wherein the surface (222) of the header coupling (200) directly engages and is fixedly secured to the surface (312) of the helix shaft (302) at the sealed joint (308).
